# EUROPEAN PATENT SPECIFICATION

(11) **EP 3 954 388 B1**
(45) Date of publication and mention of the grant of the patent: **07.10.2026**
(21) Application number: 21186316.2
(22) Date of filing: 18.08.2016
(51) Int. Cl.: A61K 39/395, C07K 16/28, A61P 35/02, C07D 403/12, C07D 473/16

(54) **COMBINATIONS AND USES THEREOF**
KOMBINATIONEN UND VERWENDUNGEN DAVON
COMBINAISONS ET LEURS UTILISATIONS

(30) Priority: 21.08.2015 EP 15181925
(43) Date of publication of application: 16.02.2022
(62) Document number(s) of the earlier application(s) in accordance with Art. 76 EPC: 16756997.9 / 3 337 506
(73) Proprietor: Incyte Corporation, Wilmington, DE 19803 (US)
(72) Inventor: ENDELL, Jan, 80333 München (DE); WINDERLICH, Mark, 81373 München (DE); BOXHAMMER, Rainer, 85653 Aying (DE)
(74) Representative: Carpmaels & Ransford LLP

(56) References cited:
- RICHARD J. RICKLES, THOMAS P. GIORDANO, SHAKIRA F. COTARD, JILL M. GRENIER, ANGELA ROMANELLI, TI CAI: "4765: Drug synergies observed for antibody and toxin components of SAR3419 ADC contribute to overall conjugate efficacy and can be combination drug or tumor cell line dependent", CANCER RESEARCH, UNIVERSITY OF CHICAGO PRESS, vol. 79, no. 19, Suppl. 1, 4765, 1 October 2014 (2014-10-01) - 9 April 2014 (2014-04-09), pages 4765, XP008178877, ISSN: 0008-5472, DOI: 10.1158/1538-7445.AM2014-4765

## Description

### Field of the Invention

The present disclosure is related to a pharmaceutical combination of an anti-CD19 antibody and a phosphoinositide 3-kinase inhibitor for the treatment of non-Hodgkin's lymphoma, chronic lymphocytic leukemia and/or acute lymphoblastic leukemia.

### Background

B cells are lymphocytes that play a large role in the humoral immune response. They are produced in the bone marrow of most mammals, and represent 5-15% of the circulating lymphoid pool. The principal function of B cells is to make antibodies against various antigens, and are an essential component of the adaptive immune system.

Because of their critical role in regulating the immune system, disregulation of B cells is associated with a variety of disorders, such as lymphomas, and leukemias. These include non-Hodgkin's lymphoma ("NHL"), chronic lymphocytic leukemia ("CLL") and acute lymphoblastic leukemia ("ALL").

NHL is a heterogeneous malignancy originating from lymphocytes. In the United States (U.S.), the incidence is estimated at 65,000/year with mortality of approximately 20,000 (American Cancer Society, 2006; and SEER Cancer Statistics Review). The disease can occur in all ages, the usual onset begins in adults over 40 years, with the incidence increasing with age. NHL is characterized by a clonal proliferation of lymphocytes that accumulate in the lymph nodes, blood, bone marrow and spleen, although any major organ may be involved. The current classification system used by pathologists and clinicians is the World Health Organization (WHO) Classification of Tumours, which organizes NHL into precursor and mature B-cell or T-cell neoplasms. The PDQ is currently dividing NHL as indolent or aggressive for entry into clinical trials. The indolent NHL group is comprised primarily of follicular subtypes, small lymphocytic lymphoma, MALT (mucosa-associated lymphoid tissue), and marginal zone; indolent encompasses approximately 50% of newly diagnosed B-cell NHL patients. Aggressive NHL includes patients with histologic diagnoses of primarily diffuse large B cell (DLBL, DLBCL, or DLCL) (40% of all newly diagnosed patients have diffuse large cell), Burkitt's, and mantle cell. The clinical course of NHL is highly variable. A major determinant of clinical course is the histologic subtype. Most indolent types of NHL are considered to be incurable disease. Patients respond initially to either chemotherapy or antibody therapy and most will relapse. Studies to date have not demonstrated an improvement in survival with early intervention. In asymptomatic patients, it is acceptable to "watch and wait" until the patient becomes symptomatic or the disease pace appears to be accelerating. Over time, the disease may transform to a more aggressive histology. The median survival is 8 to 10 years, and indolent patients often receive 3 or more treatments during the treatment phase of their disease. Initial treatment of the symptomatic indolent NHL patient historically has been combination chemotherapy. The most commonly used agents include: cyclophosphamide, vincristine and prednisone (CVP); or cyclophosphamide, adriamycin, vincristine, prednisone (CHOP). Approximately 70% to 80% of patients will respond to their initial chemotherapy, duration of remissions last on the order of 2-3 years. Ultimately the majority of patients relapse. The discovery and clinical use of the anti-CD20 antibody, rituximab, has provided significant improvements in response and survival rate. The current standard of care for most patients is rituximab + CHOP (R-CHOP) or rituximab + CVP (R-CVP). Interferon is approved for initial treatment of NHL in combination with alkylating agents, but has limited use in the U.S. Rituximab therapy has been shown to be efficacious in several types of NHL, and is currently approved as a first line treatment for both indolent (follicular lymphoma) and aggressive NHL (diffuse large B cell lymphoma). However, there are significant limitations of anti-CD20 monoclonal antibody (mAb), including primary resistance (50% response in relapsed indolent patients), acquired resistance (50% response rate upon re-treatment), rare complete response (2% complete resonse rate in relapsed population), and a continued pattern of relapse. Finally, many B cells do not express CD20, and thus many B-cell disorders are not treatable using anti-CD20 antibody therapy.

In addition to NHL there are several types of leukemias that result from disregulation of B cells. CLL is a type of adult leukemia caused by an abnormal accumulation of B lymphocytes. In CLL, the malignant lymphocytes may look normal and mature, but they are not able to cope effectively with infection. CLL is the most common form of leukemia in adults. Men are twice as likely to develop CLL as women. However, the key risk factor is age. Over 75% of new cases are diagnosed in patients over age 50. More than 10,000 cases are diagnosed every year and the mortality is almost 5,000 a year (American Cancer Society, 2006; and SEER Cancer Statistics Review). CLL is an incurable disease but progresses slowly in most cases. Many people with CLL lead normal and active lives for many years. Because of its slow onset, early-stage CLL is generally not treated since it is believed that early CLL intervention does not improve survival time or quality of life. Instead, the condition is monitored over time. Initial CLL treatments vary depending on the exact diagnosis and the progression of the disease. There are dozens of agents used for CLL therapy. Combination chemotherapy regimens such as FCR (fludarabine, cyclophosphamide and rituximab), and BR (Idelalisib and rituximab) are effective in both newly-diagnosed and relapsed CLL. Allogeneic bone marrow (stem cell) transplantation is rarely used as a first-line treatment for CLL due to its risk.

Another type of leukemia is ALL is also known as acute lymphocytic leukemia. ALL is characterised by the overproduction and continuous multiplication of malignant and immature white blood cells (also known as lymphoblasts) in the bone marrow. 'Acute' refers to the undifferentiated, immature state of the circulating lymphocytes ("blasts"), and that the disease progresses rapidly with life expectancy of weeks to months if left untreated. ALL is most common in childhood with a peak incidence of 4-5 years of age. Children of age 12- 16 die more easily from it than others. Currently, at least 80% of childhood ALL are considered curable. Under 4,000 cases are diagnosed every year and the mortality is almost 1,500 a year (American Cancer Society, 2006; and SEER Cancer Statistics Review).

The human CD 19 molecule is a structurally distinct cell surface receptor expressed on the surface of human B cells, including, but not limited to, pre-B cells, B cells in early development {i.e., immature B cells), mature B cells through terminal differentiation into plasma cells, and malignant B cells. CD 19 is expressed by most pre-B acute lymphoblastic leukemias (ALL), non-Hodgkin's lymphomas, B cell chronic lymphocytic leukemias (CLL), pro-lymphocytic leukemias, hairy cell leukemias, common acute lymphocytic leukemias, and some Null-acute lymphoblastic leukemias (Nadler et al, J. Immunol., 131 :244-250 (1983), Loken et al, Blood, 70:1316-1324 (1987), Uckun et al, Blood, 71 :13- 29 (1988), Anderson et al, 1984. Blood, 63:1424-1433 (1984), Scheuermann, Leuk. Lymphoma, 18:385-397(1995)). The expression of CD 19 on plasma cells further suggests it may be expressed on differentiated B cell tumors such as multiple myeloma, plasmacytomas, Waldenstrom's tumors (Grossbard et al., Br. J. Haematol, 102:509- 15(1998); Treon et al, Semin. Oncol, 30:248-52(2003)).

Therefore, the CD 19 antigen is a target for immunotherapy in the treatment of non-Hodgkin's lymphoma (including each the subtypes described herein), chronic lymphocytic leukemia and/or acute lymphoblastic leukemia.

Certain CD19 therapies have been shown. T cells expressing an anti-CD19 chimeric antigen receptor (CAR) including both CD3-ζ and the 4-BB costimulatory domain were administered to three patients with advanced CLL. Kalos et al., T cells with Chimeric Antigen Receptors Have Potent Antitumor Effects and Can Establish Memory in Patients with Advanced Leukemia, Science Translational Medicine, vol. 3, no. 95 (10 August 2011). Sadelain et al., The promise and potential pitfalls of chimeric antigen receptors, Current Opinion in Immunology, Elsevier, vol. 21, no.2, 2 April 2009, also describes anti-CD19 chimeric antigen receptors (CARs).

The use of a CD19 antibody in non-specific B cell lymphomas is discussed in WO2007076950 (US2007154473).

The use of a CD19 antibody in CLL, NHL and ALL is described in Scheuermann et al., CD19 Antigen in Leukemia and Lymphoma Diagnosis and Immunotherapy, Leukemia and Lymphoma, Vol. 18, 385-397 (1995).

Additional antibodies specific for CD19 are described in WO2005012493 (US7109304), WO2010053716 (US12/266,999) (Immunomedics); WO2007002223 (US US8097703) (Medarex); WO2008022152 (12/377,251) and WO2008150494 (Xencor), WO2008031056 (US11/852,106) (Medimmune); WO 2007076950 (US 11/648,505 ) (Merck Patent GmbH); WO 2009/052431 (US12/253,895) (Seattle Genetics); and WO2010095031 (12/710,442) (Glenmark Pharmaceuticals), WO2012010562 and WO2012010561 (International Drug Development), WO2011147834 (Roche Glycart), and WO 2012/156455 (Sanofi).

Combinations of antibodies specific for CD19 and other agents are described in WO2010151341 (US 13/377,514) (The Feinstein Institute); US5686072 (University of Texas), and WO2002022212 (PCT/US01/29026) (IDEC Pharmaceuticals), WO2013/024097 (14/126,928) (MorphoSys AG) and WO2013/024095 (14/127,217) (MorphoSys AG). Abstract 4765 of the AACR Annual Meeting 2014, April 5-9, 2014 in San Diego, CA, titled Drug synergies observed for antibody and toxin components of SAR3419 ADC contribute to overall conjugate efficacy and can be combination drug or tumor cell line dependent discloses the SAR3419 anti-CD19 antibody drug conjugate (ADC) with PI3K inhibitors in certain cell lines.

Certain Phosphoinositide 3-kinase inhibitors are commercially available. Idelalisib, also known as GS-1101 or CAL-101, is marketed by Gilead and has a trade name Zydelig in the United States. Idelalisib is described in US Patent Nos: 6,800,620; 8,865,730; 8,980,901; RE44599; and RE44638.

It is clear that in spite of the recent progress in the discovery and development of anti-cancer agents, many forms of cancer involving CD19-expressing tumors still have a poor prognosis. Thus, there is a need for improved methods for treating such forms of cancer.

### Summary

Neither alone nor in combination does the prior art suggest the synergistic effects of the combination of the exemplified antibody and Idelalisib in the treatment of non-Hodgkin's lymphoma, chronic lymphocytic leukemia and/or acute lymphoblastic leukemia.

The invention provides a combination comprising an antibody specific for CD19 wherein said antibody comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) and a phosphoinositide 3-kinase inhibitor for use in the treatment of non-Hodgkin's lymphoma, chronic lymphocytic leukemia and/or acute lymphoblastic leukemia.

The invention also provides an antibody specific for CD19 wherein said antibody comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) for use in combination with a phosphoinositide 3-kinase inhibitor in the treatment of non-Hodgkin's lymphoma, chronic lymphocytic leukemia and/or acute lymphoblastic leukemia.

The invention also provides a phosphoinositide 3-kinase inhibitor for use in combination with an antibody specific for CD19 wherein said antibody comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) in the treatment of non-Hodgkin's lymphoma, chronic lymphocytic leukemia and/or acute lymphoblastic leukemia.

In one aspect, the present disclosure relates to a synergistic combination of an antibody specific for CD19 and a phosphoinositide 3-kinase inhibitor. Such combinations are useful in the treatment of B cell malignancies, such as, non-Hodgkin's lymphoma (NHL), chronic lymphocytic leukemia and/or acute lymphoblastic leukemia (ALL).

*In vitro* models are considered indicative of how a certain compound or combination of compounds would behave in humans. Multiple cell lines were tested, for example, MEC-1 cells (DSMZ# ACC497) a chronic B-cell leukemia cell line. MEC-1 cells in this *in vitro* model are indicative of how the combination will work in the treatment of chronic lymphoid leukemia (CLL) in humans.

In addition, when compounds are combined in vitro, one expects that the combination has only additive effects. Surprisingly, the inventors found that the combination of a particular antibody specific for CD19 and Idelalisib mediated a synergistic level of specific cell killing *in vitro* in comparison to the antibody and Idelalisib alone.

Specifically, the inventors found that the combination of MOR00208 and Idelalisib mediated a synergistic level of specific cell killing *in vitro* in MEC-1 cells compared to the antibody and Idelalisib alone.

In addition, and also unexpectedly, the inventors found that the combination of a particular antibody specific for CD19 and Idelalisib had certain superior functional properties, in comparison to the antibody and Idelalisib alone.

In summary, the combination of the exemplified anti-CD19 antibody and Idelalisib behaved synergistically in models relevant to CLL. As CLL is a B cell related disorders and CD19 is highly expressed on B-cells, the exemplified combination would have the same mechanism of action and should also behave synergistically in the treatment of other B cell related disorders, e.g. NHL and ALL.

Therefore, the combination of the exemplified antibody specific for CD19 and Idelalisib should be effective in the treatment of humans in non-Hodgkin's lymphoma, chronic lymphocytic leukemia and/or acute lymphoblastic leukemia. The expected efficacy of the combination of the antibody specific to CD19 exemplified and Idelalisib will be confirmed in clinical trials.

As the mechanism of action of Idelalisib and other phosphoinositide 3-kinase inhibitors are similar, as they work by inhibiting one or more of the phosphoinositide 3-kinase enzymes, which are part of the PI3K/AKT/mTOR pathway, an important signalling pathway for many cellular functions such as growth control, metabolism and translation initiation, it is believed that synergy should also be seen when treating humans having non-Hodgkin's lymphoma, chronic lymphocytic leukemia and/or acute lymphoblastic leukemia with a combination of the exemplified anti-CD19 antibody and a phosphoinositide 3-kinase inhibitor other than Idelalisib.

As the exemplified anti-CD19 antibody and other anti-CD19 antibodies bind CD19, it is believed that synergy should also be seen when treating humans having non-Hodgkin's lymphoma, chronic lymphocytic leukemia and/or acute lymphoblastic leukemia with a combination of any anti-CD19 antibody and a phosphoinositide 3-kinase inhibitor, e.g., Idelalisib.

An aspect of the present disclosure comprises a synergistic combination wherein the antibody specific for CD19 comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) and Idelalisib. In preferred aspects, the combination is used for the treatment of non-Hodgkin's lymphoma, chronic lymphocytic leukemia and/or acute lymphoblastic leukemia.

### Description of Drawings

**Figure 1** shows the amino acid sequence of the variable domains of MOR00208.
**Figure** 2 shows the amino acid sequence of the Fc regions of MOR00208.
**Figures 3-6** show ADCC dose response curves of the combination of MOR00208 and Idelalisib in MEC-1 cells from four independent experiments.
**Figures 7-10** show the CI curves of the combination of MOR00208 and Idelalisib at differing doses from four independent experiments.

### Detailed description of the invention

"Synergy", "synergism" or "synergistic" mean more than the expected additive effect of a combination. The "synergy", "synergism" or "synergistic" effect of a combination is determined herein by the methods of Chou et al., Clarke et al. and/or Webb et al. See Ting-Chao Chou, Theoretical Basis, Experimental Design, and Computerized Simulation of Synergism and Antagonism in Drug Combination Studies, Pharmacol Rev 58:621-681 (2006). See also Clarke et al., Issues in experimental design and endpoint analysis in the study of experimental cytotoxic agents in vivo in breast cancer and other models, Breast Cancer Research and Treatment 46:255-278 (1997). See also Webb, J. L. (1963) Enzyme and Metabolic Inhibitors, Academic Press, New York.

The term "antibody" means monoclonal antibodies, including any isotype, such as, IgG, IgM, IgA, IgD and IgE. An IgG antibody is comprised of two identical heavy chains and two identical light chains that are joined by disulfide bonds. Each heavy and light chain contains a constant region and a variable region. Each variable region contains three segments called "complementarity-determining regions" ("CDRs") or "hypervariable regions", which are primarily responsible for binding an epitope of an antigen. They are referred to as CDR1, CDR2, and CDR3, numbered sequentially from the N-terminus. The more highly conserved portions of the variable regions outside of the CDRs are called the "framework regions". An "antibody fragment" means an Fv, scFv, dsFv, Fab, Fab' F(ab')2 fragment, or other fragment, which contains at least one variable heavy or variable light chain, each containing CDRs and framework regions.

A "phosphoinositide 3-kinase inhibitor" is a class of medical drug that functions by inhibiting one or more of the phosphoinositide 3-kinase enzymes, which are part of the PI3K/AKT/mTOR pathway, an important signalling pathway for many cellular functions such as growth control, metabolism and translation initiation.

There are a number of different classes and isoforms of PI3Ks. Class 1 PI3Ks have a catalytic subunit known as p110, with four types (isoforms) - p110 alpha, p110 beta, p110 gamma and p110 delta. Current inhibitors being studied inhibit one or more isoforms of the class I PI3Ks.

Phosphoinositide 3-kinase inhibitors include at least Idelalisib, Duvelisib and Copanlisib.

Idelalisib is marketed by Gilead Sciences, Inc. (trade name Zydelig, also named GS-1101 or CAL-101). Idelalisib is is currently labelled for the treatment of relapsed chronic lymphocytic leukemia (CLL), in combination with rituximab, in patients for whom rituximab alone would be considered appropriate therapy due to other co-morbidities; relapsed follicular B-cell non-Hodgkin lymphoma (FL) in patients who have received at least two prior systemic therapies; relapsed small lymphocytic lymphoma (SLL) in patients who have received at least two prior systemic therapies. The substance acts as a phosphoinositide 3-kinase inhibitor; more specifically, it blocks P110δ, the delta isoform of the enzyme phosphoinositide 3-kinase. The formula of Idelalisib is:

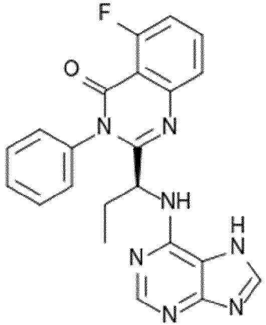

Duvelisib (IPI-145, INK1197) is a novel and selective PI3K δ/γ (delta and gamma) inhibitor. The formula of Duvelisib is:

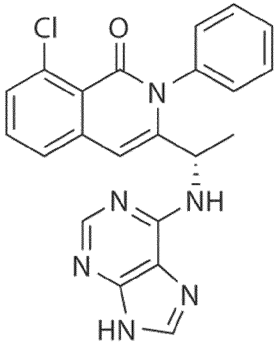

Copanlisib (BAY 80-6946), developed by Bayer, is a selective Class I phosphoinositide 3-kinase inhibitor. The formula of Copanlisib is:

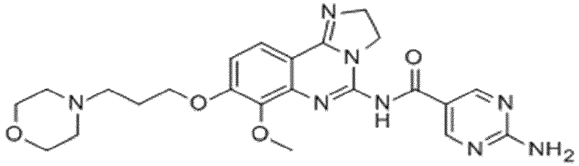

"VH" refers to the variable region of an immunoglobulin heavy chain of an antibody, or antibody fragment. "VL" refers to the variable region of the immunoglobulin light chain of an antibody, or antibody fragment.

The term "CD19" refers to the protein known as CD19, having the following synonyms: B4, B-lymphocyte antigen CD19, B-lymphocyte surface antigen B4, CVID3, Differentiation antigen CD19, MGC12802, and T-cell surface antigen Leu-12.

Human CD19 has the amino acid sequence of:










. (SEQ ID NO: 7)

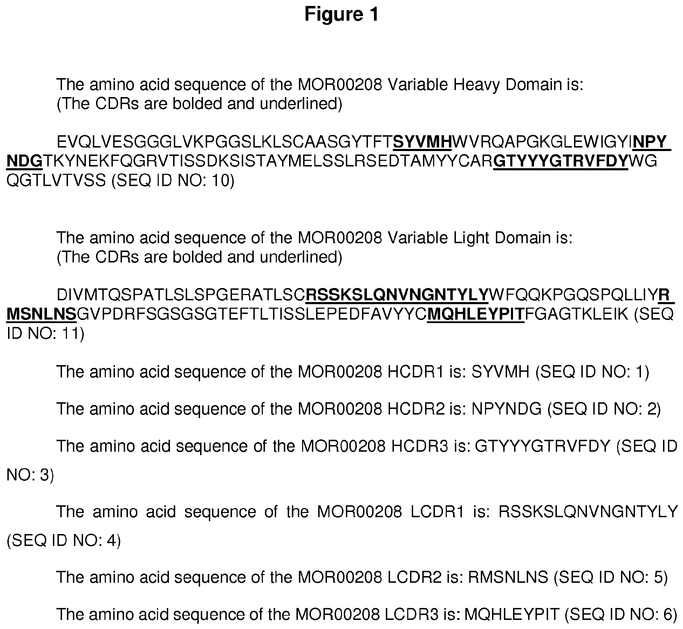

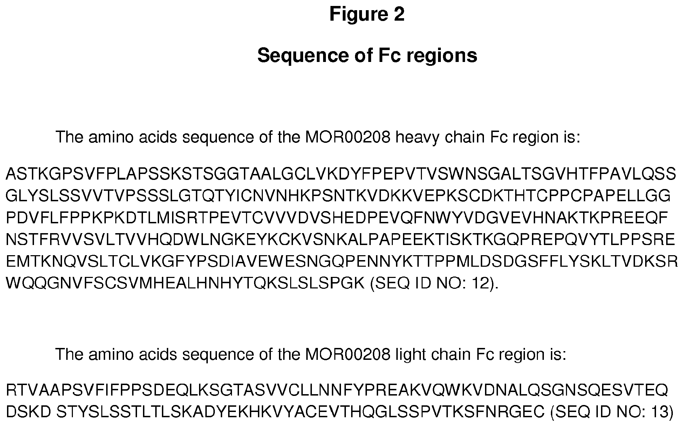

"MOR00208" is an anti-CD19 antibody. The amino acid sequence of the variable domains is provided in Figure 1. The amino acid sequence of the heavy and light chain Fc regions of MOR00208 are provided in Figure 2. "MOR00208," "XmAb 5574," and "MOR208" are used as synonyms to describe the antibody shown in Figures 1 and 2. The MOR00208 antibody is described in US patent publication no. 2010-0272723 A1 , wherein the full light chain is SEQ ID NO: 106 and the full heavy chain is SEQ ID NO: 87.

MOR00208 has been studied in human clinical trials in ALL, NHL, CLL, and Small Lymphocytic Lymphoma (SLL).

Additional antibodies specific for CD19 are described in US patent no. 7,109,304 (Immunomedics); US patent publication no. 2009-0142349 A1 (Medarex); US patent publication no. 2008-0138336 A1(Medimmune); US patent publication no. 2007-0154473 A1(Merck Patent GmbH); US patent no. 7,968,687 (Seattle Genetics); and US patent publication no. 2010-0215651 A1 (Glenmark Pharmaceuticals).

"Fc region" means the constant region of an antibody, which in humans may be of the IgG1, 2, 3, 4 subclass or others. The sequences of human Fc regions are available at IMGT, Human IGH C-REGIONs, http://www.imgt.org/IMGTrepertoire/Proteins /protein/human/IGH/IGHC/Hu_IGHCallgenes.html (retrieved on 16 May 2011).

"RefmAb33" is an antibody whose amino acid sequence is as follows:
Heavy chain including the Fc region: 
QVTLRESGPALVKPTQTLTLTCTFSGFSLSTAGMSVGWIRQPPGKALEWLADIWWDDKKH
YNPSLKDRLTISKDTSKNQVVLKVTNMDPADTATYYCARDMIFNFYFDVWGQGTTVTVSSASTKG
PSVFPLAPSSKSTSGGTAALGCLVKDYFPEPVTVSWNSGALTSGVHTFPAVLQSSGLYSLSSVVTV
PSSSLGTQTYICNVNHKPSNTKVDKKVEPKSCDKTHTCPPCPAPELLGGPDVFLFPPKPKDTLMIS
RTPEVTCVVVDVSHEDPEVQFNWYVDGVEVHNAKTKPREEQFNSTFRVVSVLTVVHQDWLNGKE
YKCKVSNKALPAPEEKTISKTKGQPREPQVYTLPPSREEMTKNQVSLTCLVKGFYPSDIAVEWESN
GQPENNYKTTPPMLDSDGSFFLYSKLTVDKSRWQQGNVFSCSVMHEALHNHYTQKSLSLSPGK
(SEQ ID NO: 8)
Light chain including the Fc region:

DIQMTQSPSTLSASVGDRVTITCSASSRVGYMHWYQQKPGKAPKLLIYDTSKLASGVPSRF
SGSGSGTEFTLTISSLQPDDFATYYCFQGSGYPFTFGGGTKVEIKRTVAAPSVFIFPPSDEQLKSGT
ASVVCLLNNFYPREAKVQWKVDNALQSGNSQESVTEQDSKDSTYSLSSTLTLSKADYEKHKVYAC
EVTHQGLSSPVTKSFNRGEC (SEQ ID NO: 9)

RefmAb33 is specific for RSV, and is used as isotype control, as it shares the same Fc region as MOR00208.

A "combination" means more than one item, e.g. a compound such as an antibody and Idelalisib .

The present disclosure also relates to combinations, pharmaceuticals, and pharmaceutical compositions containing the described combinations. The two components of the synergistic combination of the present invention, e.g. the antibody specific for CD19 and Idelalisib, may be administered together, simultaneously, separately or subsequently, either physically or in time.

Idelalisib is currently administered 150mg orally twice daily. MOR00208 is currently administered intravenously, and is currently dosed either once a week or once every two weeks. In an embodiment, Idelalisib, is administered prior to the administration of the antibody specific for CD19, e.g. MOR00208. In an embodiment, Idelalisib, is administered after the administration of the antibody specific for CD19, e.g. MOR00208.

Preferably, administration of both drugs allows for both drugs to be active in the patient at the same time. For example, if MOR00208 is dosed weekly and Idelalisib is dosed daily then the active substance of both drugs is desireably present in the patient at the same time even if both drugs are not always administered both on the same day.

"Simultaneously" or "administered together" means that the two components are administered at a time where both components (drugs) are active in the patient at the same time. It is implied by "synergism" that both drugs are active in the patient at the same time. It is not necessary for "simultaneously" or "administered together" to mean that the drugs are administered at the exact same time or always on the same day.

The two components may be formulated in different pharmaceutical compositions.

A pharmaceutical composition includes an active agent, eg. an antibody for therapeutic use in humans. A pharmaceutical composition may include acceptable carriers or excipients.

"Administered" or "administration" includes but is not limited to delivery by an injectable form, and includes, for example, intravenous, intramuscular, intradermal, topical, transdermally, intraperitoneally, intraorbitally, by implantation, or subcutaneous route or mucosal route, for example, as a nasal spray or aerosol for inhalation or as an ingestable solution, or orally, as a capsule or tablet.

A "therapeutically effective amount" of a compound or combination refers to an amount able to effect a measurable improvement, alleviate or partially arrest the clinical manifestations of a given disease or disorder. The amount that is effective for a particular therapeutic purpose will depend on the severity of the disease or injury as well as on the weight and general state of the subject. It will be understood that determination of an appropriate dosage may be achieved, using routine experimentation, by constructing a matrix of values and testing different points in the matrix, all of which is within the ordinary skills of a trained physician or clinical scientist.

The "CDRs" herein are defined by either Chothia et al or Kabat et al. See Chothia C, Lesk AM. (1987) Canonical structures for the hypervariable regions of immunoglobulins. J Mol Biol., 196(4):901-17. See Kabat E.A, Wu T.T., Perry H.M., Gottesman K.S. and Foeller C. (1991). Sequences of Proteins of Immunological Interest. 5th edit., NIH Publication no. 91-3242, US Dept. of Health and Human Services, Washington, DC.

"Cross competes" means the ability of an antibody or other binding agent to interfere with the binding of other antibodies or binding agents to CD19 in a standard competitive binding assay. The ability or extent to which an antibody or other binding agent is able to interfere with the binding of another antibody or binding molecule to CD19, and, therefore whether it can be said to cross-compete according to the invention, can be determined using standard competition binding assays. One suitable assay involves the use of the Biacore technology (e.g. by using the BIAcore 3000 instrument (Biacore, Uppsala, Sweden)), which can measure the extent of interactions using surface plasmon resonance technology. Another assay for measuring cross-competing uses an ELISA-based approach. A high throughput process for "epitope binning" antibodies based upon their cross-competition is described in International Patent Application No. WO 2003/48731

The term "epitope" includes any protein determinant capable of specific binding to an antibody or otherwise interacting with a molecule. Epitopic determinants generally consist of chemically active surface groupings of molecules such as amino acids or carbohydrate or sugar side chains and can have specific three-dimensional structural characteristics, as well as specific charge characteristics. An epitope may be "linear" or "conformational." The term "linear epitope" refers to an epitope with all of the points of interaction between the protein and the interacting molecule (such as an antibody) occur linearally along the primary amino acid sequence of the protein (continuous). The term "conformational epitope" refers to an epitope in which discontinuous amino acids that come together in three dimensional conformation. In a conformational epitope, the points of interaction occur across amino acid residues on the protein that are separated from one another.

"Binds the same epitope as" means the ability of an antibody or other binding agent to bind to CD19 and having the same epitope as the exemplified antibody. The epitopes of the exemplified antibody and other antibodies to CD19 can be determined using standard epitope mapping techniques. Epitope mapping techniques, well known in the art. include Epitope Mapping Protocols in Methods in Molecular Biology, Vol. 66 (Glenn E.Morris, Ed., 1996) Humana Press, Totowa, New Jersey. For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Patent No. 4,708,871 ; Geysen et al, (1984) Proc. Natl. Acad. Sci. USA 8:3998-4002; Geysen et al, (1985) Proc. Natl. Acad. Sci. USA 82:78-182; Geysen et al, (1986) Mol. Immunol. 23 :709-715. Similarly, conformational epitopes are readily identified by determining spatial conformation of amino acids such as by, e.g., hydrogen/deuterium exchange, x-ray crystallography and two-dimensional nuclear magnetic resonance. See, e.g., Epitope Mapping Protocols, supra. Antigenic regions of proteins can also be identified using standard antigenicity and hydropathy plots, such as those calculated using, e.g., the Omiga version 1.0 software program available from the Oxford Molecular Group. This computer program employs the Hopp/Woods method, Hopp et al, (1981) Proc. Natl. Acad. Sci USA 78:3824-3828; for determining antigenicity profiles, and the Kyte-Doolittle technique, Kyte et al, (1982) J.Mol. Biol. 157: 105-132; for hydropathy plots.

### Embodiments

An aspect of the present disclosure comprises a combination of an antibody specific for CD19 and a phosphoinositide 3-kinase inhibitor for use in the treatment of non-Hodgkin's lymphoma, chronic lymphocytic leukemia and/or acute lymphoblastic leukemia. In embodiments, the combination is synergistic.

Herein, the combination of the exemplified anti-CD19 antibody and Idelalisib behaved synergistically in *in vitro* models relevant to CLL. As CLL is a B cell related disorder and CD19 is highly expressed on B-cells, the exemplified combination should have the same mechanism of action and should also behave synergistically in the treatment of other B cell related disorders, e.g. NHL and ALL. Therefore, the combination of the exemplified antibody specific for CD19 and Idelalisib should be effective in the treatment of humans in non-Hodgkin's lymphoma, chronic lymphocytic leukemia and/or acute lymphoblastic leukemia. The expected efficacy of the combination of the antibody specific to CD19 exemplified and Idelalisib will be confirmed in clinical trials.

MEC-1 cells (DSMZ# ACC497), a chronic B-cell leukemia cell line, were tested.

Addititional cell lines are evaluated: Ramos cells (ATCC number CRL-1596), a human Burkitt's lymphoma cells. HG-3 (DSMZ#ACC765), and CII (DSMZ#ACC773) are a chronic lymphocytic leukemia cell line. Su-DHL 6 (DSMZ#ACC572), and U2932 (DSMZ#ACC633) are a Diffuse large B-cell lymphoma (DLBCL) cell line. JVM-2 (ATCC^{®} CRL-3002) is a mantle cell lymphoma cell line. BALL-1 (DSMZ#ACC742) is an acute lymphoblastic leukemia cell line.

MEC-1 cells in this in vitro model are indicative of how the combination will work in the treatment of chronic lymphoid leukemia (CLL) in humans. Ramos cells in this in vitro model are indicative of how the combination will work in the treatment of non-Hogkins lymphoma (NHL) in humans. HG-3 and CII cells in this in vitro model are indicative of how the combination will work in the treatment of chronic lymphoid leukemia (CLL) in humans. Su-DHL 6 and U2932 cells in this in vitro model are indicative of how the combination will work in the treatment non-Hodgkin's lymphoma in humans. JVM-2 cells in this in vitro model are indicative of how the combination will work in the treatment non-Hodgkin's lymphoma in humans. BALL-1 cells in this in vitro model are indicative of how the combination will work in the treatment of acute lymphoblastic leukemia in humans.

The Chou index and Clarke et al. values indicate clear synergism of the combination of MOR00208 and Idelalisib in the specific killing of MEC-1 cells as compared to MOR00208 and Idelalisib alone.

In summary, the combination of the exemplified anti-CD19 antibody and Idelalisib behaved synergistically in models relevant to CLL. Therefore, the combination of the exemplified antibody specific for CD19 and Idelalisib should be effective in the treatment of humans in non-Hodgkin's lymphoma, chronic lymphocytic leukemia and/or acute lymphoblastic leukemia.

As the mechanism of action of Idelalisib and other phosphoinositide 3-kinase inhibitors are similar, as they all work by inhibiting one or more of the phosphoinositide 3-kinase enzymes, which are part of the PI3K/AKT/mTOR pathway, an important signalling pathway for many cellular functions such as growth control, metabolism and translation initiation, it is believed that synergy should also be seen when treating humans having non-Hodgkin's lymphoma, chronic lymphocytic leukemia and/or acute lymphoblastic leukemia with a combination of the exemplified anti-CD19 antibody and a phosphoinositide 3-kinase inhibitor other than Idelalisib.

As the exemplified anti-CD19 antibody and other anti-CD19 antibodies bind CD19, it is believed that synergy should also be seen when treating humans having non-Hodgkin's lymphoma, chronic lymphocytic leukemia and/or acute lymphoblastic leukemia with a combination of any anti-CD19 antibody and a phosphoinositide 3-kinase inhibitor, where the anti-CD19 antibody is, for example, described in US patent publication no. 2010-0272723 A1(Xencor), WO2005012493, WO2010053716 (Immunomedics); WO2007002223 (Medarex); WO2008022152 (Xencor); WO2008031056 (Medimmune); WO 2007/076950 (Merck Patent GmbH); WO 2009/052431 (Seattle Genetics); and WO2010095031 (Glenmark Pharmaceuticals).

In embodiments, the antibody specific for CD19 comprises an antibody that cross-competes with the antibody comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6).

In embodiments, the antibody specific for CD19 comprises an antibody that binds to the same epitope as an antibody comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6).

In embodiments, the antibody specific for CD19 comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6).

In embodiments, the antibody specific for CD19 comprises a variable heavy chain of the sequence EVQLVESGGGLVKPGGSLKLSCAASGYTFTSYVMHWVRQAPGKGLEWIGYINPY NDGTKYNEKFQGRVTISSDKSISTAYMELSSLRSEDTAMYYCARGTYYYGTRVFDYWG QGTLVTVSS (SEQ ID NO: 10) and a variable light chain of the sequence

DIVMTQSPATLSLSPGERATLSCRSSKSLQNVNGNTYLYWFQQKPGQSPQLLIYR
MSNLNSGVPDRFSGSGSGTEFTLTISSLEPEDFAVYYCMQHLEYPITFGAGTKLEIK (SEQ ID NO:
11).

In certain embodiments said antibody comprises a heavy chain constant domain of the sequence

ASTKGPSVFPLAPSSKSTSGGTAALGCLVKDYFPEPVTVSWNSGALTSGVHTFPAVLQSSGLYSL
SSVVTVPSSSLGTQTYICNVNHKPSNTKVDKKVEPKSCDKTHTCPPCPAPELLGGPDVFLFPPKPK
DTLMISRTPEVTCVVVDVSHEDPEVQFNWYVDGVEVHNAKTKPREEQFNSTFRVVSVLTVVHQD
WLNGKEYKCKVSNKALPAPEEKTISKTKGQPREPQVYTLPPSREEMTKNQVSLTCLVKGFYPSDIA
VEWESNGQPENNYKTTPPMLDSDGSFFLYSKLTVDKSRWQQGNVFSCSVMHEALHNHYTQKSL
SLSPGK (SEQ ID NO: 12).

In embodiments, the antibody specific for CD19 comprises a light chain constant domain of the sequence


. (SEQ ID NO: 13)

In embodiments, the phosphoinositide 3-kinase inhibitor is Idelalisib.

In embodiments, the components of the combination, the antibody specific for CD19 and Idelalisib, are administered separately. In an embodiment, Idelalisib is administered prior to administration of the antibody specific for CD19. In an embodiment, Idelalisib is administered after the administration of the antibody specific for CD19. In embodiments, the components of the combination, the antibody specific for CD19 and Idelalisib, are administered simultaneously or together.

In embodiments the combination is a pharmaceutical composition. In embodiments, the composition comprises an acceptable carrier. In embodiments, the combination is administered in an effective amount.

In another aspect the synergistic combination of an antibody specific for CD19 comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) and Idelalisib is able to mediate killing of MEC-1 cells by ADCC in the presence of isolated human PBMCs with an at least two-fold, three-fold, four-fold, or five-fold better efficacy than Idelalisib alone.

An aspect of the present disclosure comprises a synergistic combination of an antibody specific for CD19 comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) and Idelalisib for the treatment of non-Hodgkin's lymphoma, chronic lymphocytic leukemia and/or acute lymphoblastic leukemia. In embodiments, the non-Hodgkin's lymphoma is selected from the group consisting of follicular lymphoma, small lymphocytic lymphoma, mucosa-associated lymphoid tissue, marginal zone, diffuse large B cell, Burkitt's, and mantle cell.

### Examples

### Example 1: Cytotoxicity of MEC-1 cells using MOR00208 and Idelalisib alone and in combination

### Materials

Cell lines MEC-1 cells (DSMZ# ACC497) chronic B-cell leukemia cell line; JVM-2 (ATCC^{®} CRL-3002) a mantle cell lymphoma cell line; Ramos cells (ATCC number CRL-1596), a human Burkitt's lymphoma cells; HG-3 (DSMZ#ACC765), and CII (DSMZ#ACC773) are a chronic lymphocytic leukemia cell line; Su-DHL 6 (DSMZ#ACC572), and U2932 (DSMZ#ACC633) are a Diffuse large B-cell lymphoma (DLBCL) cell line; and BALL-1 (DSMZ#ACC742) is an acute lymphoblastic leukemia cell line.

Culture conditions of cell lines used are according to supplier's information.

Cell Medium: Iscove's Modified Dulbecco's Medium (IMDM), Invitrogen, Cat No.: 31980-048; RPMI1640, Invitrogen, Cat No.: 31870-074; GlutaMAX, Invitrogen, CAT No.: 35050-38 LOT No.: 1654740; FCS: Sigma CAT No.: F7524 LOT No.: 111M3396.

NKs: RPMI1640, with GlutaMAXTM, Invitrogen, Cat No.: 31870-074, 10% FCS; Biocoll: Biochrome AG CAT No.: L6115 LOT No.: 0034D; MACS NK cell isolation kit: Miltenyi Biotec CAT No.: 130-092-657 LOT No.: 5150327376; Idelalisib: Selleck Chem CAT No.:S2226; FCS: Sigma CAT No.: F7524 LOT No.: 111M3396; and RefmAb33 (anti-RSV) with same Fc region as MOR00208.

### Methods

The cytotoxicity of MOR00208 and Idelalisib alone and in combination were tested in the MEC-1 cell line..

Idelalisib is a phosphoinositide 3-kinase inhibitor; more specifically, it blocks P110δ, the delta isoform of the enzyme phosphoinositide 3-kinase. Idelalisib alone has little to no cyctotoxic effects against MEC-1 cells. MOR00208 targets CD19 and mediates target cell killing via ADCC.

The following were used as controls: a) MEC-1 cells + RefmAb33 + DMSO + NK cells, b) MEC-1 cells + DMSO + NK cells, c) MEC-1 cells + DMSO.

Target cell killing was measured using the following parameters: Idelalisib at concentrations of 0.3, 1, 3 and 10 µM; MOR00208 at concentrations of 1.5, 0.015 and 0.0015 µg/ml and the combination of MOR00208 and Idelalisib at the same concentrations.

In the Idelalisib group, MOR00208 alone group and in the MOR00208+Idelalisib combination group, target cells were pre-treated with Idelalisib for 7 days prior to the ADCC assay measurements. The target cells were counted and stained using 10µM CFSE end concentration. For DMSO treated target cells, an effector : target (E:T) ratio of 2:1 is chosen, corresponding to a cell density of 5x10⁵/ml. The proliferative effect on target cells caused by Idelalisib treatment was included by adjusting the E:T ratio in inhibitor treated cells. The NK cells are counted and adjusted to 1x10⁶/ml. The target cell killing assays were performed as follows: using 96 well plates, 100µl of target cell suspension was added per well, followed by 100µl cell suspension of NK cells to each well resulting in an E:T ratio of 2:1. The antibodies were diluted in a range of 10 - 0.00001 nM (corresponding to 1.5 - 0.0000015 µg/ml) in medium. Cells were centrifuged and target : effector cell-pellets were re-suspended in 100 µl antibody-containing medium or the according control solution. The assay was incubated for 4h in CO2-incubator at 37°C. After 10 min incubation on ice, 50 µl DAPI solution was added to each well (final concentration 1 µg/ml) and incubated on ice for 10 min. The cell killing measurements were performed with FACS-Verse. Dead target cells were DAPI positive.

### Data

In total, six experiments were performed in order to determine the mediation of ADCC on MEC-1 cells by the combination of MOR00208 and Idelalisib. In two out of the six experiments, the data was excluded from analysis because the RefmAb control and DMSO alone control showed 25% higher killing compared to the MEC-1 cells only control. The autoreactivity of the NK cells prevented a proper analysis in those two experiments.

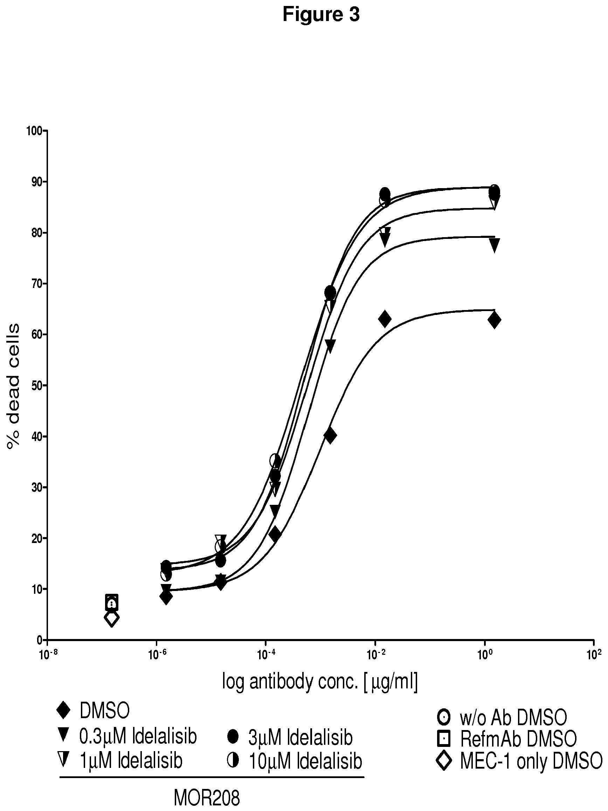

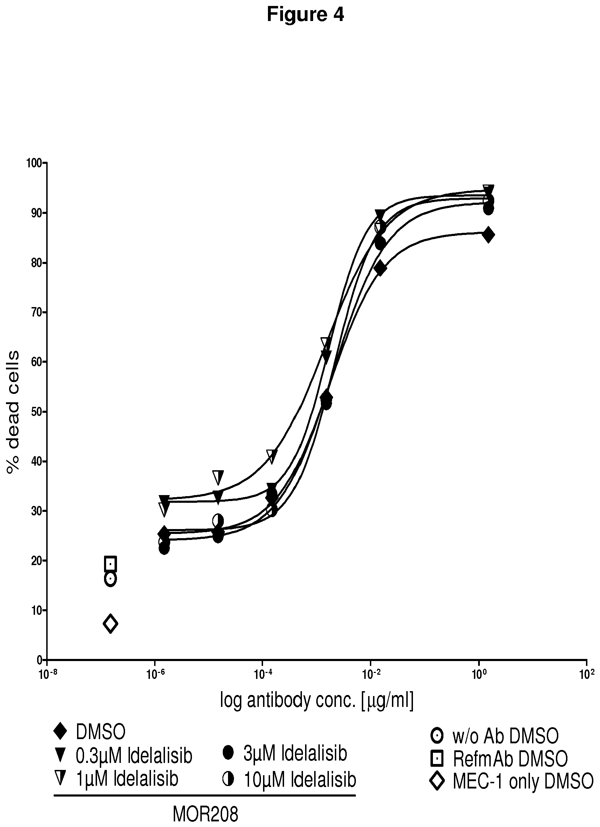

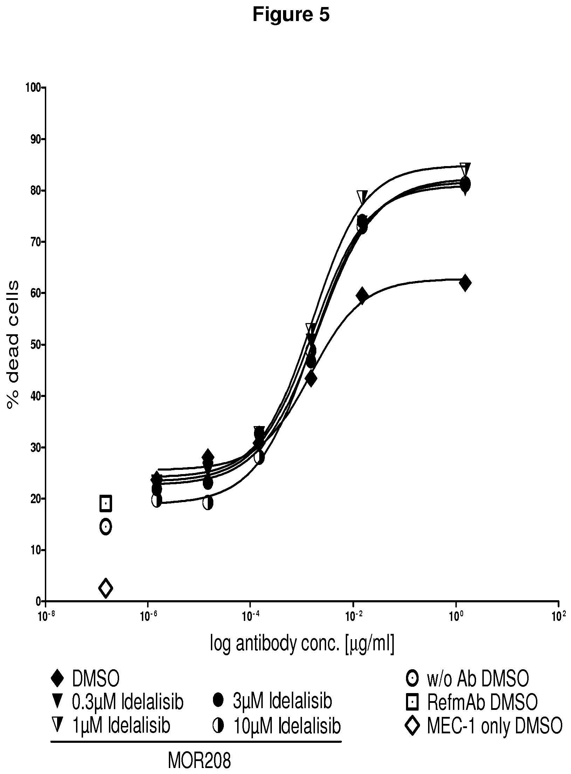

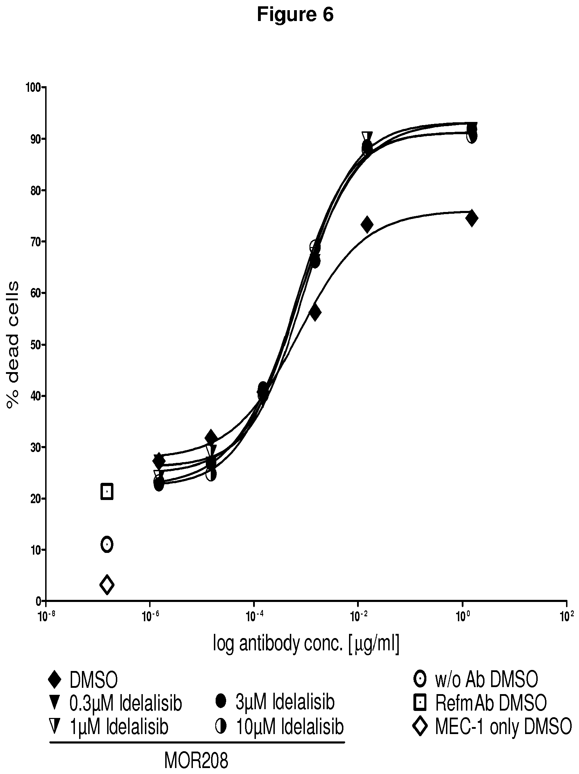

The ADCC dose response curves for Experiments 1-4 are shown in Figures 3-6.

The percent (%) dead cells (raw data) for Experiments 1-4 are shown in Tables 1-16 below.

### Experiment 1

**Table 1: Idelalisib at 10 µM**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **A: MOR00208 alone** | 62,9 | 63,1 | 40,3 |
| **B: Idela alone 10µM** | 11,6 | 11,6 | 11,6 |
| **C: control (0,03%DMSO/Ref33)** | 7,4 | 7,4 | 7,4 |
| **AB: combination** | 88,0 | 86,4 | 68,2 |

**Table 2 Idelalisib at 3 µM**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **A: MOR00208 alone** | 62,9 | 63,1 | 40,3 |
| **B: Idela alone 3µM** | 11,3 | 11,3 | 11,3 |
| **C: control (0,03%DMSO/Ref33)** | 7,4 | 7,4 | 7,4 |
| **AB: combination** | 87,7 | 87,6 | 68,1 |

**Table 3 Idelalisib at 1 µM**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **A: MOR00208 alone** | 62,9 | 63,1 | 40,3 |
| **B: Idela alone 1µM** | 10,8 | 10,8 | 10,8 |
| **C: control (0,03%DMSO/Ref33)** | 7,4 | 7,4 | 7,4 |
| **AB: combination** | 85,9 | 79,6 | 65,3 |

**Table 4 Idelalisib at 0.3 µM**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **A: MOR00208 alone** | 62,9 | 63,1 | 40,3 |
| **B: Idela alone 0.3µM** | 6,8 | 6,8 | 6,8 |
| **C: control (0,03%DMSO/Ref33)** | 7,4 | 7,4 | 7,4 |
| **AB: combination** | 77,5 | 78,5 | 57,6 |

### Experiment 2

**Table 5 Idelalisib at 10 µM**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **A: MOR00208 alone** | 85,6 | 78,9 | 52,9 |
| **B: Idela alone 10µM** | 14,7 | 14,7 | 14,7 |
| **C: control (0,03%DMSO/Ref33)** | 19,3 | 19,3 | 19,3 |
| **AB: combination** | 92,5 | 87,2 | 52,6 |

**Table 6 Idelalisib at 3 µM**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **A: MOR00208 alone** | 85,6 | 78,9 | 52,9 |
| **B: Idela alone 3µM** | 14,6 | 14,6 | 14,6 |
| **C: control (0,03%DMSO/Ref33)** | 19,3 | 19,3 | 19,3 |
| **AB: combination** | 90,9 | 83,9 | 51,8 |

**Table 7 Idelalisib at 1 µM**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **A: MOR00208 alone** | 85,6 | 78,9 | 52,9 |
| **B: Idela alone 1µM** | 20,7 | 20,7 | 20,7 |
| **C: control (0,03%DMSO/Ref33)** | 19,3 | 19,3 | 19,3 |
| **AB: combination** | 94,2 | 86,5 | 63,4 |

**Table 8 Idelalisib at 0.3 µM**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **A: MOR00208 alone** | 85,6 | 78,9 | 52,9 |
| **B: Idela alone 0.3µM** | 20,8 | 20,8 | 20,8 |
| **C: control (0,03%DMSO/Ref33)** | 19,3 | 19,3 | 19,3 |
| **AB: combination** | 93,9 | 89,3 | 60,9 |

### Experiment 3

**Table 9 Idelalisib at 10 µM**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **A: MOR00208 alone** | 62,0 | 59,5 | 43,5 |
| **B: Idela alone 10µM** | 11,5 | 11,5 | 11,5 |
| **C: control (0,03%DMSO/Ref33)** | 19,1 | 19,1 | 19,1 |
| **AB: combination** | 81,3 | 73,0 | 48,9 |
| | | | |
| | | | |

**Table 10 Idelalisib at 3 µM**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **A: MOR00208 alone** | 62,0 | 59,5 | 43,5 |
| **B: Idela alone 3µM** | 14,0 | 14,0 | 14,0 |
| **C: control (0,03%DMSO/Ref33)** | 19,1 | 19,1 | 19,1 |
| **AB: combination** | 81,1 | 74,0 | 46,8 |

**Table 11 Idelalisib at 1 µM**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **A: MOR00208 alone** | 62,0 | 59,5 | 43,5 |
| **B: Idela alone 1µM** | 18,7 | 18,7 | 18,7 |
| **C: control (0,03%DMSO/Ref33)** | 19,1 | 19,1 | 19,1 |
| **AB: combination** | 83,9 | 78,5 | 52,7 |

**Table 12 Idelalisib at 0.3 µM**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **A: MOR00208 alone** | 62,0 | 59,5 | 43,5 |
| **B: Idela alone 0.3µM** | 17,3 | 17,3 | 17,3 |
| **C: control (0,03%DMSO/Ref33)** | 19,1 | 19,1 | 19,1 |
| **AB: combination** | 80,4 | 73,7 | 50,8 |

### Experiment 4

**Table 13 Idelalisib at 10 µM**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **A: MOR00208 alone** | 74,6 | 73,3 | 56,3 |
| **B: Idela alone 10µM** | 12,1 | 12,1 | 12,1 |
| **C: control (0,03%DMSO/Ref33)** | 21,4 | 21,4 | 21,4 |
| **AB: combination** | 90,6 | 88,0 | 68,9 |

**Table 14 Idelalisib at 3 µM**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **A: MOR00208 alone** | 74,6 | 73,3 | 56,3 |
| **B: Idela alone 3µM** | 13,8 | 13,8 | 13,8 |
| **C: control (0,03%DMSO/Ref33)** | 21,4 | 21,4 | 21,4 |
| **AB: combination** | 91,9 | 88,5 | 66,2 |

**Table 15 Idelalisib at 1 µM**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **A: MOR00208 alone** | 74,6 | 73,3 | 56,3 |
| **B: Idela alone 1µM** | 15,9 | 15,9 | 15,9 |
| **C: control (0,03%DMSO/Ref33)** | 21,4 | 21,4 | 21,4 |
| **AB: combination** | 91,7 | 89,9 | 67,4 |

**Table 16 Idelalisib at 0.3 µM**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **A: MOR00208 alone** | 74,6 | 73,3 | 56,3 |
| **B: Idela alone 0.3µM** | 15,5 | 15,5 | 15,5 |
| **C: control (0,03%DMSO/Ref33)** | 21,4 | 21,4 | 21,4 |
| **AB: combination** | 90,4 | 87,7 | 66,1 |

### Calculation of synergism: Clarke et al.

Where one drug has low activity, as here, Idelalisib alone has low cytotoxity activity against MEC-1 cells, synergy can be determined by statistical evidence that the combination is significantly different from the inhibitory drug alone. See Clarke et al., Issues in experimental design and endpoint analysis in the study of experimental cytotoxic agents in vivo in breast cancer and other models, Breast Cancer Research and Treatment 46:255-278 (1997).

The % dead cells (raw data) from Tables 1-16 was analysed in the following way:

| | |
|---|---|
| Antagonistic | (AB)/C < (A/C) x (B/C) |
| Additive | (AB)/C = (A/C) x (B/C) |
| Synergistic | (AB)/C > (A/C) x (B/C) |

where A is the treatment with MOR00208 alone; B is the treatment with Idelalisib alone; C is response to the control DMSO + RefMab33; AB is the combination of treatments A and B.

### Experiment 1

**Table 17: Clarke analysis of Data shown in Table 1**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **(AB)/C** | 12,0 | 11,7 | 9,3 |
| **(A/C)x(B/C)** | 13,5 | 13,5 | 8,6 |

**Table 18 Clarke analysis of Data shown in Table 2**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **(AB)/C** | 11,9 | 11,9 | 9,3 |
| **(A/C)x(B/C)** | 13,1 | 13,1 | 8,4 |

**Table 19 Clarke analysis of Data shown in Table 3**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **(AB)/C** | 11,7 | 10,8 | 8,9 |
| **(A/C)x(B/C)** | 12,5 | 12,5 | 8,0 |

**Table 20 Clarke analysis of Data shown in Table 4**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **(AB)/C** | 10,5 | 10,7 | 7,8 |
| **(A/C)x(B/C)** | 7,9 | 7,9 | 5,0 |

### Experiment 2

**Table 21 Clarke analysis of Data shown in Table 5**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **(AB)/C** | 4,8 | 4,5 | 2,7 |
| **(A/C)x(B/C)** | 3,4 | 3,1 | 2,1 |

**Table 22 Clarke analysis of Data shown in Table 6**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **(AB)/C** | 4,7 | 4,4 | 2,7 |
| **(A/C)x(B/C)** | 3,4 | 3,1 | 2,1 |

**Table 23 Clarke analysis of Data shown in Table 7**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **(AB)/C** | 4,9 | 4,5 | 3,3 |
| **(A/C)x(B/C)** | 4,8 | 4,4 | 3,0 |

**Table 24 Clarke analysis of Data shown in Table 8**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **(AB)/C** | 4,9 | 4,6 | 3,2 |
| **(A/C)x(B/C)** | 4,8 | 4,4 | 3,0 |

### Experiment 3

**Table 25 Clarke analysis of Data shown in Table 9**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **(AB)/C** | 4,3 | 3,8 | 2,6 |
| **(A/C)x(B/C)** | 2,0 | 1,9 | 1,4 |

**Table 26 Clarke analysis of Data shown in Table 10**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **(AB)/C** | 4,2 | 3,9 | 2,5 |
| **(A/C)x(B/C)** | 2,4 | 2,3 | 1,7 |

**Table 27 Clarke analysis of Data shown in Table 11**

| | **MOR00208 Concentration** | | |
|---|---|---|---|
| | **10 nM** | **0.1 nM** | **0.01** nM |
| | **1.5 µg/ml** | **0.015 µg/ml** | **0.0015 µg/ml** |
| **(AB)/C** | 4,4 | 4,1 | 2,8 |
| **(A/C)x(B/C)** | 3,2 | 3,1 | 2,2 |

**Table 28 Clarke analysis of Data shown in Table 12**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **(AB)/C** | 4,2 | 3,9 | 2,7 |
| **(A/C)x(B/C)** | 2,9 | 2,8 | 2,1 |

### Experiment 4

**Table 29 Clarke analysis of Data shown in Table 13**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **(AB)/C** | 4,2 | 4,1 | 3,2 |
| **(A/C)x(B/C)** | 2,0 | 1,9 | 1,5 |

**Table 30 Clarke analysis of Data shown in Table 14**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **(AB)/C** | 4,3 | 4,1 | 3,1 |
| **(A/C)x(B/C)** | 2,2 | 2,2 | 1,7 |

**Table 31 Clarke analysis of Data shown in Table 15**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **(AB)/C** | 4,3 | 4,2 | 3,1 |
| **(A/C)x(B/C)** | 2,6 | 2,5 | 2,0 |

**Table 32 Clarke analysis of Data shown in Table 16**

| | **MOR00208 Concentration** | | |
|---|---|---|---|
| | **10 nM** | **0.1 nM** | **0.01 nM** |
| | **1.5 µg/ml** | **0.015 µg/ml** | **0.0015 µg/ml** |
| **(AB)/C** | 4,2 | 4,1 | 3,1 |
| **(A/C)x(B/C)** | 2,5 | 2,5 | 1,9 |

### Results

Experiments 2-4 at each concentration showed clear synergy of the combination of MOR00208 + Idelalisib using the methods of Clarke et al. Experiment 1, however, at a few concentrations did not show synergism, because the Idelalisib group (see Tables 1-3) showed a small effect, which effect was slightly greater than the control (~4% greater than the control). This small (~ 4%) difference as compared to the control, is well within the range of the other controls, so can be attributed to experimental setup.

### Calculation of synergism: Combination Index (CI)

In order to confirm the results of synergy as calculated using Clarke et al. above, the Combination Index (CI) method was applied to the % dead cells (raw data) of Tables 1-16. For CI calculations, we used 0.3, 1, 3 and 10 µM Idelalisib and three MOR208 concentrations (1.5, 0.015 and 0.0015 µg/ml).

Such calculations are described in Ting-Chao Chou, Theoretical Basis, Experimental Design, and Computerized Simulation of Synergism and Antagonism in Drug Combination Studies, Pharmacol Rev 58:621-681 (2006), and Chou TC, Talalay P, Quantitative analysis of dose-effect relationships: the combined effects of multiple drugs or enzyme inhibitors. Adv Enzyme Regul 22: 27-55 (1984). The methods of Chou-Talalay are carried out using the Cl-isobol method.

### Median effect equation

The median-effect equation models the effect of an inhibitor (such as a drug) as Fₐ/Fᵤ =(D/D50)^m, where D is the dose, Fₐ and Fᵤ is the fraction of the system affected and unaffected by the dose D (Fₐ + Fᵤ = 1); D50 is the dose producing the median effect (e.g. IC50, ED50, LD50). The constant *m* determines the shape of the dose-effect curve.
We use GraphPad Prism to carry out a nonlinear regression calculation to estimate the parameters *m* and D50.

### Cl-isobol method

The Cl-isobol method provides a quantitative assessment of synergism between drugs. A combination index (CI) is estimated from dose-effect data of single and combined drug treatments. A value of Cl less than 1 indicates synergism; CI = 1 indicates additive effect; and CI > 1 indicates antagonism. Drug interaction (synergism or antagonism) is more pronounced the farther a CI value is from 1.

Formally, the combination index (CI) of a combined drug treatment is defined as $CI = {D}_{1} / {D}_{x 1} + {D}_{2} / {D}_{X 2}$

Here D1 and D2 are the doses of drug 1 and drug 2 of the combination, respectively; and Dx1, and Dx2 is the dose of a treatment with only drug 1 and drug 2 that would give the same effect as that of the combination. The doses Dx1 and Dx2 need to be estimated from the dose-effect data of single drug treatments. Essentially, a median effect equation is fitted to the data of each drug. From the median effect equation of a drug, we can estimate the dose (i.e. D) necessary to produce an effect (i.e. Fa, Fu). The further a point lies from the additive line, the bigger the different between 1 and its CI, thus the stronger the (synergistic or antagonistic) effect is.

### Results

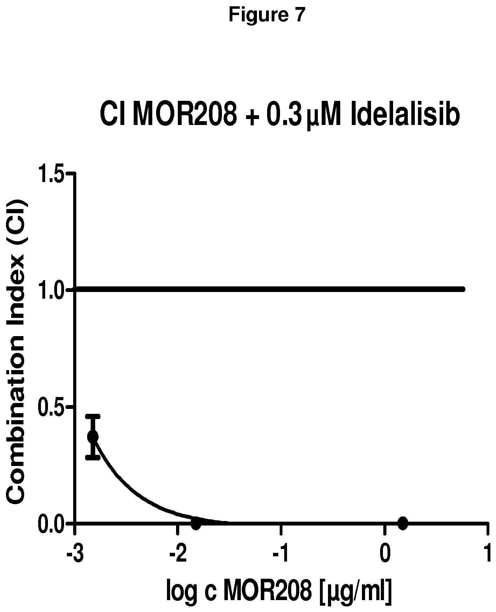

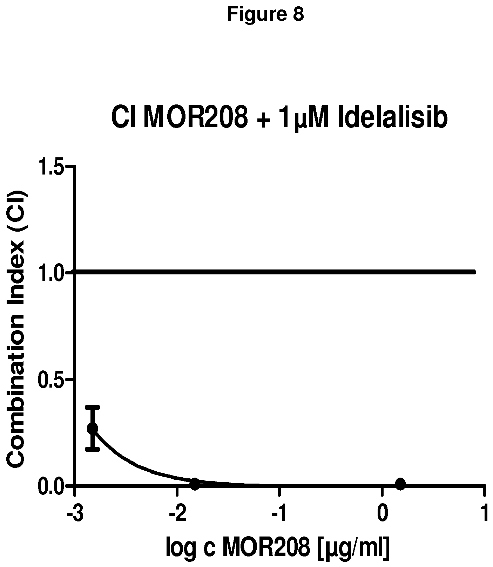

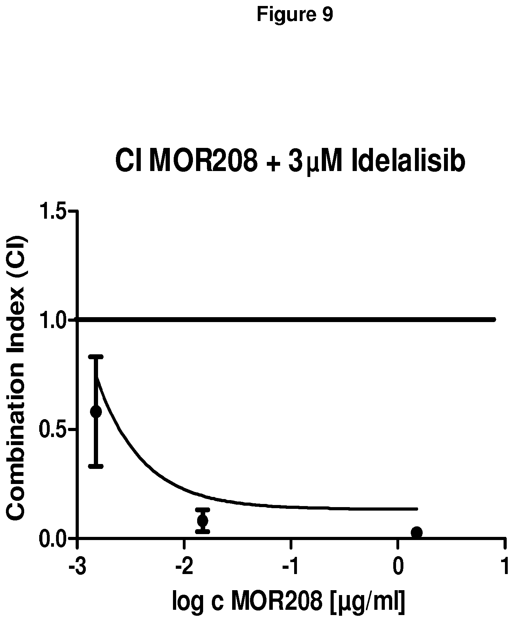

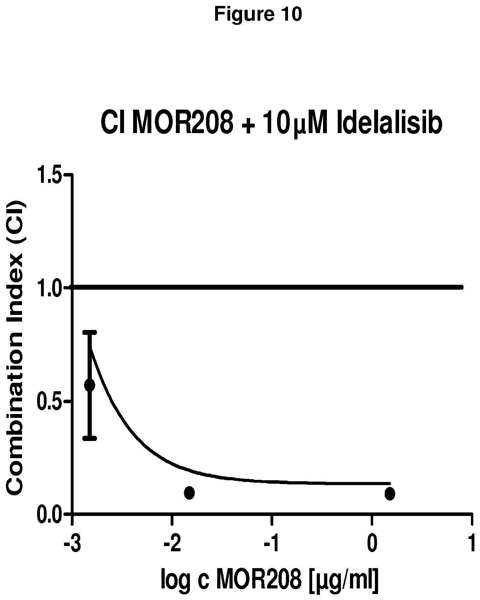

The curves generated for the Chou based synergy calculatios are shown in Figures 7-10. The Chou index values indicate clear synergism in all Experiments 1-4 of the combination of MOR00208 and Idelalisib in the specific killing of MEC-1 cells as compared to MOR00208 and Idelalisib alone.

The combination of MOR00208 and Idelalisib behaved synergistically in the MEC-1 CLL cell line. Therefore, it is believed that the combination of MOR00208 and Idelalisib is synergistic in the treatment of CLL in humans.

In addition, it is also believed that the combination of MOR00208 and Idelalisib will behave synergistically in the treatment of non-Hodgkin's lymphoma (NHL), chronic lymphoid leukemia (CLL), and acute lymphoblastic leukemia (ALL) in humans.

## Claims

1. A combination comprising an antibody specific for CD19 wherein said antibody comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) and a phosphoinositide 3-kinase inhibitor for use in the treatment of non-Hodgkin's lymphoma, chronic lymphocytic leukemia and/or acute lymphoblastic leukemia.

2. An antibody specific for CD19 wherein said antibody comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) for use in combination with a phosphoinositide 3-kinase inhibitor in the treatment of non-Hodgkin's lymphoma, chronic lymphocytic leukemia and/or acute lymphoblastic leukemia.

3. A phosphoinositide 3-kinase inhibitor for use in combination with an antibody specific for CD19 wherein said antibody comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) in the treatment of non-Hodgkin's lymphoma, chronic lymphocytic leukemia and/or acute lymphoblastic leukemia.

4. The combination for use according to any one of the preceding claims, wherein the antibody comprises a variable heavy chain of the sequence EVQLVESGGGLVKPGGSLKLSCAASGYTFTSYVMHWVRQAPGKGLEWIGYINPY NDGTKYNEKFQGRVTISSDKSISTAYMELSSLRSEDTAMYYCARGTYYYGTRVFDYW GQGTLVTVSS (SEQ ID NO: 10) and a variable light chain of the sequence

DIVMTQSPATLSLSPGERATLSCRSSKSLQNVNGNTYLYWFQQKPGQSPQLLIYR
MSNLNSGVPDRFSGSGSGTEFTLTISSLEPEDFAVYYCMQHLEYPITFGAGTKLEIK
(SEQ ID NO: 11).

5. The combination for use according to any one of the preceding claims, wherein the antibody comprises a heavy chain constant domain of the sequence

ASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAPE
LLGGPDVFLFPPKPKDTLMISRTPEVTCVVVDVSHEDPEVQFNWYVDGVEVHNAKTKP
REEQFNSTFRVVSVLTVVHQDWLNGKEYKCKVSNKALPAPEEKTISKTKGQPREPQV
YTLPPSREEMTKNQVSLTCLVKGFYPSDIAVEWESNGQPENNYKTTPPMLDSDGSFFL
YSKLTVDKSRWQQGNVFSCSVMHEALHNHYTQKSLSLSPGK (SEQ ID NO: 12).

6. The combination for use according to any one of the preceding claims, wherein the antibody comprises a light chain constant domain of the sequence


 (SEQ ID NO:
13).

7. The combination for use according to any one of the preceding claims, wherein said antibody specific for CD19 and said phosphoinositide 3-kinase inhibitor are formulated in different pharmaceutical compositions.

8. The combination for use according to any one of the preceding claims, wherein the phosphoinositide 3-kinase inhibitor is Idelalisib.

9. The combination for use according to any one of the preceding claims, wherein the phosphoinositide 3-kinase inhibitor is Duvelisib.

10. The combination for use according to any one of the preceding claims, wherein the phosphoinositide 3-kinase inhibitor is Copanlisib.

11. The combination for use according to any one of the preceding claims wherein the treatment is of non-Hodgkin's lymphoma, optionally wherein the non-Hodgkin's lymphoma is selected from the group consisting of follicular lymphoma, small lymphocytic lymphoma, mucosa-associated lymphoid tissue lymphoma, marginal zone lymphoma, diffuse large B cell lymphoma, Burkitt's lymphoma, and mantle cell lymphoma.

12. The combination for use according to any one of claims 1-10, wherein the treatment is of chronic lymphocytic leukemia.

13. The combination for use according to any one of claims 1-10, wherein the treatment is of acute lymphoblastic leukemia.

## Patentansprüche

1. Kombination, umfassend einen für CD19 spezifischen Antikörper, wobei der Antikörper eine HCDR1-Region der Sequenz SYVMH (SEQ ID NO: 1), eine HCDR2-Region der Sequenz NPYNDG (SEQ ID NO: 2), eine HCDR3-Region der Sequenz GTYYYGTRVFDY (SEQ ID NO: 3), eine LCDR1-Region der Sequenz RSSKSLQNVNGNTYLY (SEQ ID NO: 4), eine LCDR2-Region der Sequenz RMSNLNS (SEQ ID NO: 5) und eine LCDR3-Region der Sequenz MQHLEYPIT (SEQ ID NO: 6) umfasst, sowie einen Phosphoinositid-3-Kinase-Inhibitor, zur Verwendung bei der Behandlung von Non-Hodgkin-Lymphom, chronischer lymphatischer Leukämie und/oder akuter lymphoblastischer Leukämie.

2. Antikörper, der für CD19 spezifisch ist, wobei der Antikörper eine HCDR1-Region der Sequenz SYVMH (SEQ ID NO: 1), eine HCDR2-Region der Sequenz NPYNDG (SEQ ID NO: 2), eine HCDR3-Region der Sequenz GTYYYGTRVFDY (SEQ ID NO: 3), eine LCDR1-Region der Sequenz RSSKSLQNVNGNTYLY (SEQ ID NO: 4), eine LCDR2-Region der Sequenz RMSNLNS (SEQ ID NO: 5) und eine LCDR3-Region der Sequenz MQHLEYPIT (SEQ ID NO: 6) umfasst, zur Verwendung in Kombination mit einem Phosphoinositid-3-Kinase-Inhibitor bei der Behandlung von Non-Hodgkin-Lymphom, chronischer lymphatischer Leukämie und/oder akuter lymphoblastischer Leukämie.

3. Phosphoinositid-3-Kinase-Inhibitor zur Verwendung in Kombination mit einem für CD19 spezifischen Antikörper, wobei der Antikörper eine HCDR1-Region der Sequenz SYVMH (SEQ ID NO: 1), eine HCDR2-Region der Sequenz NPYNDG (SEQ ID NO: 2), eine HCDR3-Region der Sequenz GTYYYGTRVFDY (SEQ ID NO: 3), eine LCDR1-Region der Sequenz RSSKSLQNVNGNTYLY (SEQ ID NO: 4), eine LCDR2-Region der Sequenz RMSNLNS (SEQ ID NO: 5) und eine LCDR3-Region der Sequenz MQHLEYPIT (SEQ ID NO: 6) umfasst, bei der Behandlung von Non-Hodgkin-Lymphom, chronischer lymphatischer Leukämie und/oder akuter lymphoblastischer Leukämie.

4. Kombination zur Verwendung nach einem der vorhergehenden Ansprüche, wobei der Antikörper eine variable schwere Kette der Sequenz EVQLVESGGGLVKPGGSLKLSCAASGYTFTSYVMHWVRQAPGKGLEWIGYINPY NDGTKYNEKFQGRVTISSDKSISTAYMELSSLRSEDTAMYYCARGTYYYGTRVFD YW GQGTLVTVSS (SEQ ID NO: 10) und eine variable leichte Kette der Sequenz DIVMTQSPATLSLSPGERATLSCRSSKSLQNVNGNTYLYWFQQKPGQSPQLLIYR MSNLNSGVPDRFSGSGSGTEFTLTISSLEPEDFAVYYCMQHLEYPITFGAGTKLE IK (SEQ ID NO: 11) umfasst.

5. Kombination zur Verwendung nach einem der vorhergehenden Ansprüche, wobei der Antikörper eine Schwere-Kette-konstante-Domäne der Sequenz ASTKGPSVFPLAPSSKSTSGGTAALGCLVKDYFPEPVTVSWNSGALTSGVHTFPA VLQ SSGLYSLSSVVTVPSSSLGTQTYICNVNHKPSNTKVDKKVEPKSCDKTHTCPPCP APE LLGGPDVFLFPPKPKDTLMISRTPEVTCVVVDVSHEDPEVQFNWYVDGVEVHNAK TKP REEQFNSTFRVVSVLTVVHQDWLNGKEYKCKVSNKALPAPEEKTISKTKGQPREP QV YTLPPSREEMTKNQVSLTCLVKGFYPSDIAVEWESNGQPENNYKTTPPMLDSDGS FFL YSKLTVDKSRWQQGNVFSCSVMHEALHNHYTQKSLSLSPGK (SEQ ID NO: 12) umfasst.

6. Kombination zur Verwendung nach einem der vorhergehenden Ansprüche, wobei der Antikörper eine Leichte-Kette-konstante-Domäne der Sequenz RTVAAPSVFIFPPSDEQLKSGTASVVCLLNNFYPREAKVQWKVDNALQSGNSQES VTE QDSKDSTYSLSSTLTLSKADYEKHKVYACEVTHQGLSSPVTKSFNRGEC (SEQ ID NO: 13) umfasst.

7. Kombination zur Verwendung nach einem der vorhergehenden Ansprüche, wobei der für CD19 spezifische Antikörper und der Phosphoinositid-3-Kinase-Inhibitor in verschiedenen pharmazeutischen Zusammensetzungen formuliert sind.

8. Kombination zur Verwendung nach einem der vorhergehenden Ansprüche, wobei es sich bei dem Phosphoinositid-3-Kinase-Inhibitor um Idelalisib handelt.

9. Kombination zur Verwendung nach einem der vorhergehenden Ansprüche, wobei es sich bei dem Phosphoinositid-3-Kinase-Inhibitor um Duvelisib handelt.

10. Kombination zur Verwendung nach einem der vorhergehenden Ansprüche, wobei es sich bei dem Phosphoinositid-3-Kinase-Inhibitor um Copanlisib handelt.

11. Kombination zur Verwendung nach einem der vorhergehenden Ansprüche, wobei Non-Hodgkin-Lymphom behandelt wird, gegebenenfalls wobei das Non-Hodgkin-Lymphom aus der Gruppe ausgewählt ist, die aus follikulärem Lymphom, kleinzelligem lymphozytischem Lymphom, MALT(Mucosa-Associated Lymphoid Tissue)-Lymphom, Marginalzonen-Lymphom, diffusem großzelligem B-Zell-Lymphom, Burkitt-Lymphom und Mantelzell-Lymphom besteht.

12. Kombination zur Verwendung nach einem der Ansprüche 1-10, wobei chronische lymphatische Leukämie behandelt wird.

13. Kombination zur Verwendung nach einem der Ansprüche 1-10, wobei akute lymphoblastische Leukämie behandelt wird.

## Revendications

1. Combinaison comprenant un anticorps spécifique de CD19, ledit anticorps comprenant une région HCDR1 de séquence SYVMH (SEQ ID NO: 1), une région HCDR2 de séquence NPYNDG (SEQ ID NO: 2), une région HCDR3 de séquence GTYYYGTRVFDY (SEQ ID NO: 3), une région LCDR1 de séquence RSSKSLQNVNGNTYLY (SEQ ID NO: 4), une région LCDR2 de séquence RMSNLNS (SEQ ID NO: 5), et une région LCDR3 de séquence MQHLEYPIT (SEQ ID NO: 6) et un inhibiteur de phosphoinositide 3-kinase pour utilisation dans le traitement du lymphome non hodgkinien, de la leucémie lymphocytaire chronique et/ou de la leucémie lymphoblastique aiguë.

2. Anticorps spécifique de CD19, ledit anticorps comprenant une région HCDR1 de séquence SYVMH (SEQ ID NO: 1), une région HCDR2 de séquence NPYNDG (SEQ ID NO: 2), une région HCDR3 de séquence GTYYYGTRVFDY (SEQ ID NO: 3), une région LCDR1 de séquence RSSKSLQNVNGNTYLY (SEQ ID NO: 4), une région LCDR2 de séquence RMSNLNS (SEQ ID NO: 5), et une région LCDR3 de séquence MQHLEYPIT (SEQ ID NO: 6) pour utilisation en combinaison avec un inhibiteur de phosphoinositide 3-kinase dans le traitement du lymphome non hodgkinien, de la leucémie lymphocytaire chronique et/ou de la leucémie lymphoblastique aiguë.

3. Inhibiteur de phosphoinositide 3-kinase pour utilisation en combinaison avec un anticorps spécifique de CD19, ledit anticorps comprenant une région HCDR1 de séquence SYVMH (SEQ ID NO: 1), une région HCDR2 de séquence NPYNDG (SEQ ID NO: 2), une région HCDR3 de séquence GTYYYGTRVFDY (SEQ ID NO: 3), une région LCDR1 de séquence RSSKSLQNVNGNTYLY (SEQ ID NO: 4), une région LCDR2 de séquence RMSNLNS (SEQ ID NO: 5), et une région LCDR3 de séquence MQHLEYPIT (SEQ ID NO: 6) dans le traitement du lymphome non hodgkinien, de la leucémie lymphocytaire chronique et/ou de la leucémie lymphoblastique aiguë.

4. Combinaison pour utilisation selon l'une quelconque des revendications précédentes, dans lequel l'anticorps comprend une chaîne lourde variable de la séquence EVQLVESGGGLVKPGGSLKLSCAASGYTFTSYVMHWVRQAPGKGLEWIGYINPY NDGTKYNEKFQGRVTISSDKSISTAYMELSSLRSEDTAMYYCARGTYYYGTRVFD YW GQGTLVTVSS (SEQ ID NO: 10) et une chaîne légère variable de la séquence

DIVMTQSPATLSLSPGERATLSCRSSKSLQNVNGNTYLYWFQQKPGQSPQLLIYR
MSNLNSGVPDRFSGSGSGTEFTLTISSLEPEDFAVYYCMQHLEYPITFGAGTKLE
IK (SEQ ID NO: 11).

5. Combinaison pour utilisation selon l'une quelconque des revendications précédentes, dans laquelle l'anticorps comprend un domaine constant de chaîne lourde de la séquence

ASTKGPSVFPLAPSSKSTSGGTAALGCLVKDYFPEPVTVSWNSGALTSGVHTFPA
VLQ
SSGLYSLSSVVTVPSSSLGTQTYICNVNHKPSNTKVDKKVEPKSCDKTHTCPPCP
APE
LLGGPDVFLFPPKPKDTLMISRTPEVTCVVVDVSHEDPEVQFNWYVDGVEVHNAK
TKP
REEQFNSTFRVVSVLTVVHQDWLNGKEYKCKVSNKALPAPEEKTISKTKGQPREP
QV
YTLPPSREEMTKNQVSLTCLVKGFYPSDIAVEWESNGQPENNYKTTPPMLDSDGS
FFL YSKLTVDKSRWQQGNVFSCSVMHEALHNHYTQKSLSLSPGK (SEQ ID
NO: 12).

6. Combinaison pour utilisation selon l'une quelconque des revendications précédentes, dans laquelle l'anticorps comprend un domaine constant de chaîne légère de la séquence



(SEQ ID NO: 13).

7. Combinaison pour utilisation selon l'une quelconque des revendications précédentes, dans laquelle ledit anticorps spécifique de CD19 et ledit inhibiteur de phosphoinositide 3-kinase sont formulés dans des compositions pharmaceutiques différentes.

8. Combinaison pour utilisation selon l'une quelconque des revendications précédentes, dans laquelle l'inhibiteur de phosphoinositide 3-kinase est l'idélalisib.

9. Combinaison pour utilisation selon l'une quelconque des revendications précédentes, dans laquelle l'inhibiteur de phosphoinositide 3-kinase est le duvélisib.

10. Combinaison pour utilisation selon l'une quelconque des revendications précédentes, dans laquelle l'inhibiteur de phosphoinositide 3-kinase est le copanlisib.

11. Combinaison pour utilisation selon l'une quelconque des revendications précédentes, le traitement étant du lymphome non hodgkinien, éventuellement le lymphome non hodgkinien étant choisi dans le groupe constitué par le lymphome folliculaire, le lymphome lymphocytaire à petites cellules, le lymphome du tissu lymphoïde associé aux muqueuses, le lymphome de la zone marginale, le lymphome diffus à grandes cellules B, le lymphome de Burkitt et le lymphome à cellules du manteau.

12. Combinaison pour utilisation selon l'une quelconque des revendications 1 à 10, le traitement étant une leucémie lymphocytaire chronique.

13. Combinaison pour utilisation selon l'une quelconque des revendications 1 à 10, le traitement étant une leucémie lymphoblastique aiguë.
